(19) Europäisches Patentamt
European Patent Office
Office européen des brevets

(11) EP 0 665 014 B1

(12) **EUROPÄISCHE PATENTSCHRIFT**

(45) Veröffentlichungstag und Bekanntmachung des Hinweises auf die Patenterteilung:
**03.09.1997 Patentblatt 1997/36**

(51) Int. Cl.$^6$: **A61K 31/495**, C07D 487/08

(21) Anmeldenummer: **95100953.9**

(22) Anmeldetag: **25.01.1995**

(54) **3-Benzoyl-3,7-diazabicyclo(3,3,1)nonan-Verbindungen mit anti-arrhythmischer Wirkung**
3-benzoyl-3,7-diazabicyclo(3,3,1)nonane anti-arrhythmic compounds
Dérivés de 3-benzoyl-3,7-diazabicyclo(3,3,1)nonane présentant une activité antiarrhythmique

(84) Benannte Vertragsstaaten:
**AT BE CH DE DK ES FR GB GR IE IT LI LU NL PT SE**

(30) Priorität: **01.02.1994 DE 4402933**

(43) Veröffentlichungstag der Anmeldung:
**02.08.1995 Patentblatt 1995/31**

(73) Patentinhaber: **Solvay Pharmaceuticals GmbH**
**30173 Hannover (DE)**

(72) Erfinder:
- **Schön, Uwe, Dr.**
  **D-31303 Burgdorf (DE)**
- **Brückner, Reinhard, Dr.**
  **D-30559 Hannover (DE)**
- **Meil, Jörg, Dr.**
  **D-30173 Hannover (DE)**
- **Thormählen, Dirk, Dr.**
  **D-31039 Rheden (DE)**

(74) Vertreter: **Lauer, Dieter, Dr.**
**Solvay Pharmaceuticals GmbH,**
**Hans-Böckler-Allee 20**
**30173 Hannover (DE)**

(56) Entgegenhaltungen:

| | |
|---|---|
| **EP-A- 0 103 833** | **EP-A- 0 306 871** |
| **EP-A- 0 308 843** | **DE-A- 2 658 558** |
| **DE-A- 3 112 055** | |

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 665 014 B1

Printed by Rank Xerox (UK) Business Services
2.14.12/3.4

## Beschreibung

Die vorliegende Erfindung betrifft die Verwendung von 3,7,9,9-tetrasubstituierten 3,7-Diazabicyclo[3,3,1]nonan-Verbindungen, welche in 3-Stellung einen gegebenenfalls substituierten Benzoylrest tragen, zur Behandlung von Herzrhythmusstörungen und zur Herstellung von für diese Behandlung geeigneten Arzneimitteln.

Der Erfindung liegt die Aufgabe zugrunde, neue antiarrhythmisch wirksame pharmazeutische Zubereitungen mit verbessertem Wirkungsprofil zu entwickeln.

Aus der EP-A-0 103 833 sind 3,7,9,9-tetraalkylierte 3,7-Diazabicyclo[3,3.1]nonan-Verbindungen bekannt, welche antiarrhythmische und herzfrequenzsenkende Wirkungen besitzen. Aus der EP-A-0 306 871 sind in 3-Stellung durch Cinnamyl- oder Benzhydrylreste substituierte 7,9,9-trialkylierte 3,7-Diazabicyclo[3,3,1]nonan-Verbindungen bekannt, welche herzfrequenzsenkende Wirkungen und daneben geringe antiarrhythmische Eigenschaften besitzen. Aus der EP-A-0 308 843 und der DE-OS 31 112 055 sind 3-Benzoyl-3,7-diazabicyclo[3,3, 1]nonan-Verbindungen bekannt, welche in 7-Stellung ebenfalls aromatisch substituiert sind und in 9-Stellung unsubstituiert oder durch funktionelle Gruppen tragende Reste substituiert sind und welche antiarrhythmische Eigenschaften besitzen.

Aus der DE-OS 26 58 558 sind 3-Alkanoyl-und 3-Aroyl-3,7-diazabicyclo[3,3,1]nonan-Derivate bekannt, für welche zentral analgetische Wirkungen angegeben werden.

Es wurde nun gefunden, daß eine Gruppe von unter den Umfang der DE-OS 26 58 558 fallenden 7,9,9-trialkylierten 3-Benzoyl-3,7-diazabicyclo[3,3,1] nonan-Verbindungen ein antiarrhythmisches Wirkprofil zeigen, welches sie zur Behandlung von Herzrhythmusstörungen, insbesondere tachykarden Arrhythmien, geeignet macht.

Erfindungsgemäß werden als Wirkstoffe zur Herstellung von antiarrhythmisch wirksamen pharmazeutischen Zubereitungen zur Behandlung von Herzrhythmusstörungen in größeren Säugetieren und Menschen 3-Benzoyl-3,7-diazabicyclo[3,3,1]nonan-Verbindungen der allgemeinen Formel I
(siehe Formel I) worin

| | |
|---|---|
| $R^1$ | eine Alkylgruppe mit 1 - 6 Kohlenstoffatomen oder eine Cycloalkylalkylgruppe mit 4 - 7 Kohlenstoffatomen bedeutet, |
| $R^2$ | Alkyl mit 1-4 Kohlenstoffatomen bedeutet und |
| $R^3$ | Alkyl mit 1-4 Kohlenstoffatomen bedeutet oder |
| $R^2$ und $R^3$ | gemeinsam eine Alkylenkette mit 3 - 6 Kohlenstoffatomen bilden, |
| $R^4$ | für Wasserstoff, Halogen, Cyano, Nitro, Trifluormethyl oder die $R^6$-$SO_2$-Gruppe, worin $R^6$ Fluor oder niederes Alkyl mit 1 bis 4 Kohlenstoffatomen bedeutet, steht und |
| $R^5$ | Wasserstoff, Halogen, Trifluormethyl oder Nitro bedeutet, |

und deren physiologisch verträgliche Säureadditionssalze verwendet.

Sofern in den Verbindungen der Formel I $R^1$ für eine Alkylgruppe steht, kann diese geradkettig oder verzweigt sein und 1 - 6, vorzugsweise 3 - 5 Kohlenstoffatome enthalten. Eine Cycloalkylalkylgruppe $R^1$ kann 4 - 7 Kohlenstoffatome enthalten. Als besonders geeignete Reste $R^1$ haben sich Alkylreste mit 3 - 5 Kohlenstoffatomen erwiesen.

Sofern die Substituenten $R^2$ und $R^3$ niederes Alkyl darstellen, können diese Alkylgruppen geradkettig oder verzweigt sein und 1 - 4, vorzugsweise 1 - 3, Kohlenstoffatome enthalten und insbesondere Methyl bedeuten. Sofern $R^2$ und $R^3$ gemeinsam eine Alkylengruppe bilden, kann diese 3 - 6, vorzugsweise 4 - 5, Kohlenstoffatome enthalten. Insbesondere haben sich solche Verbindungen, worin $R^2$ und $R^3$ gemeinsam eine Alkylenkette mit 4 - 5 Kohlenstoffatomen bilden, als geeignet erwiesen. Der Substituent $R^4$ des Benzoylrestes steht vorzugsweise für Halogen. Sofern $R^4$ für eine $R^6$-$SO_2$-Gruppe steht, kann eine darin enthaltene niedere Alkylgruppe $R^6$ 1 - 4 Kohlenstoffatome enthalten und insbesondere Methyl darstellen.

Als physiologisch verträgliche Säureadditionssalze der Verbindungen der Formel I eignen sich Salze mit anorganischen Säuren, z.B. Halogenwasserstoffsäuren, insbesondere Chlorwasserstoffsäure, oder Schwefelsäure oder mit organischen Säuren, beispielsweise niederen aliphatischen Mono- oder Dicarbonsäuren wie Essigsäure, Malonsäure, Fumarsäure, Weinsäure, Milchsäure, Maleinsäure oder Zitronensäure oder aromatischen Carbonsäuren wie z.B. Salicylsäure oder auch organischen Sulfonsäuren wie beispielsweise niederen Alkylsulfonsäuren wie Methansulfonsäure oder gegebenenfalls im Benzolring durch Halogen oder niederes Alkyl substituierten Benzolsulfonsäuren wie p-Toluolsulfonsäure.

Die erfindungsgemäß zur Behandlung von Herzrhythmusstörungen eingesetzten Verbindungen der Formel I fallen unter den Umfang der in der DE-OS 26 58 558 beschriebenen 3-Aroyl-3,7-diazabicyclo[3,3,1]nonan-Verbindungen mit zentralanalgetischen Wirkungen und sind teilweise aus dieser DE-OS bekannt.

Es wurde nun überraschenderweise gefunden, daß die erfindungsgemäß verwendete Gruppe der Verbindungen der Formel I und ihre physiologisch verträglichen Säureadditionssalze antiarrhythmische Wirkungen besitzen. Sie zeigen insbesondere Klasse III antiarrhythmische Eigenschaften und bewirken eine Verlängerung der effektiven Refraktärzeit im Herzen, welche zu einer Verlängerung des QT-Intervalls im EKG führt. Die Verbindungen besitzen ein günstiges Wirkprofil mit guter Verträglichkeit, langer Wirkungsdauer und einer so hohen Selektivität der antiarrhythmischen Wirkung gegenüber bradycarden und blutdrucksenkenden Eigenschaften, daß im antiarrhythmisch wirksamen Dosisbereich keine therapeutisch unerwünschte Beeinflußung der Herzfrequenz und/oder des Blutdrucks auftreten. Die Verbindungen zeichnen sich dadurch aus, daß die antiarrhythmische Wirkung unter tachycarden Bedingungen besonders gut ausgeprägt ist.

Die antiarrhythmischen Wirkungen der Verbindungen lassen sich in pharmakologischen Standardtestmethoden nachweisen.

Beschreibung der pharmakologischen Untersuchungsmethoden:

1. Bestimmung der minimalen toxischen Dosis

Männlichen Mäusen von 20 bis 25 g Gewicht werden p.o. Maximaldosen von 300 mg/kg der Testsubstanz verabreicht. Die Tiere werden 3 Stunden lang sorgfältig auf Toxizitätssymptome beobachtet. Über einen Zeitraum von 72 Stunden nach der Applikation werden zusätzlich alle Symptome und Todesfälle registriert. Begleitsymptome werden ebenfalls beobachtet und registriert. Wenn Tod oder starke toxische Symptome beobachtet werden, werden weiteren Mäusen zunehmend geringere Dosen verabreicht, bis keine toxischen Symptome mehr auftreten. Die niedrigste Dosis, welche Tod oder starke toxische Symptome hervorruft, wird in der nachfolgenden Tabelle A als minimale toxische Dosis angegeben. Die in Tabelle A angegebenen Beispielnummern beziehen sich auf die nachstehenden Herstellungsbeispiele.

Tabelle A

| Testsubstanz Beispiel Nr. | Minimale toxische Dosis mg/kg Maus p.o. |
|---|---|
| 4 | > 300 |
| 6 | > 300 |
| 8 | > 300 |
| 15 | > 300 |
| 17 | > 300 |
| 18 | > 300 |
| 20 | > 300 |

2. In vivo Untersuchung der antiarrhythmischen Eigenschaften der Substanzen unter tachycarden Bedingungen an narkotisierten Meerschweinchen.

Die Wirkungen der Substanzen auf die effektive Refraktärzeit (= ERP) und den Blutdruck bei i.v.-Applikation bei erhöhter Herzfrequenz wurden an narkotisierten Meerschweinchen untersucht. Den Tieren wurde unter Vollnarkose ein bipolarer Reizkatheter über eine Jugularvene in den rechten Ventrikel geschoben. Über diesen wurde die Herzfrequenz der Tiere durch elektrische Reizung während der gesamten Untersuchung auf ca. 150 % ihrer normalen Herzfrequenz gehalten. In die andere Jugularvene wurde eine Kanüle zur i.v.-Applikation der Testsubstanzen eingeführt. Während der Untersuchung wurden der systolische und der diastolische arterielle Blutdruck (= SAP und DAP) in einer Arteria carotis über einen Druckmesser (Statham-Drucktransducer) gemessen. Die Testsubstanzen wurden i.v. in steigenden Dosen (kumulativ) appliziert. Vor Applikation der ersten Dosis und jeweils 8 Minuten nach jeder Dosisgabe wurde die ERP mittels eines Doppelpulsprotokolls bestimmt. Die Dosis, bei der eine Verlängerung der ERP auf 115 % des Ausgangswertes erreicht wurde, wurde als effektive Dosis (= $ERP\text{-}ED_{115}$) berechnet. Als effektive Dosen für eine blutdrucksenkende Wirkung wurden die Dosis, bei der der SAP auf 85 % seines Ausgangswertes gesenkt wurde (= $SAP\text{-}ED_{85}$), und die Dosis, bei welcher der DAP auf 85 % seines Ausgangswertes gesenkt wurde (= $DAP\text{-}ED_{85}$), berechnet.

In der nachfolgenden Tabelle B werden die mit der vorstehend beschriebenen Methode erhaltenen Ergebnisse wiedergegeben. Die für die Testsubstanzen angegebenen Beispielsnummern beziehen sich auf die nachstehenden Herstellungsbeispiele.

Tabelle B

| Beispiel Nr. | antiarrhythmische Wirkung ERP-$ED_{115}$ in µmol/kg i.v. | Blutdrucksenkung $ED_{85}$ in µmol/kg i.v. | |
|---|---|---|---|
| | | DAP | SAP |
| 4 | 2 | > 32 | > 32 |
| 7 | 3,2 | 4 | 5 |

Die die Refraktärzeit verlängernde Wirkung der Substanzen läßt sich auch in in vitro Tests durch Bestimmung der funktionellen Refraktärzeit am isolierten Papillarmuskel der rechten Herzkammer von Meerschweinchen nachweisen.

Die vorstehenden Testergebnisse zeigen, daß die Verbindungen der Formel I antiarrhythmische Wirkungen besitzen und die effektive Refraktärzeit des Herzmuskels deutlich verlängern und daß eine effektive blutdrucksenkende Wirkung der Substanzen erst bei Dosen auftritt, die erheblich höher als die für die Refraktärzeitverlängerung effektiven Dosen sind.

Aufgrund ihres vorstehend beschriebenen Wirkungsprofils eignen sich die Substanzen zur Unterdrückung von tachycarden Herzrhythmusstörungen (Extrasystolen, Kammerflattern und - flimmern) und können zur Prophylaxe und Behandlung von Herzrhythmusstörungen in größeren Säugetieren und Menschen Verwendung finden. Insbesondere sind die Substanzen geeignet, das Auftreten von Tachyarrhythmien, d.h. Arrhythmien, die mit einem Anstieg der Herzfrequenz gekoppelt sind, zu verhindern.

Die zu verwendenden Dosen können individuell verschieden sein und variieren naturgemäß je nach Art des zu behandelnden Zustandes und der verwendeten Substanz und der Applikationsform. Zum Beispiel werden parenterale Formulierungen im allgemeinen weniger Wirkstoff enthalten als orale Präparate. Im allgemeinen eignen sich zur Applikation am Menschen und an größeren Säugetieren jedoch Arzneiformen mit einem Wirkstoffgehalt von 0,5 bis 100, insbesondere 1 bis 25, mg Wirkstoff pro Einzeldosis.

Die Verbindungen können erfindungsgemäß zusammen mit üblichen pharmazeutischen Hilfs- und/oder Trägerstoffen in festen oder flüssigen pharmazeutischen Zubereitungen enthalten sein. Als Beispiel fester Präparate seien oral applizierbare Präparate wie Tabletten, Dragees, Kapseln, Pulver oder Granulate genannt oder auch Suppositorien. Diese Präparate können pharmazeutisch übliche anorganische und/oder organische Trägerstoffe wie z.B. Talkum, Milchzucker oder Stärke neben pharmazeutisch üblichen Hilfsmitteln, beispielsweise Gleitmitteln oder Tablettensprengmitteln, enthalten. Es können zusätzlich übliche pharmazeutische Hilfsmittel, beispielsweise Gleitmittel oder Tablettensprengmittel, enthalten sein. Flüssige Präparate wie Lösungen, Suspensionen oder Emulsionen der Wirkstoffe können die üblichen Verdünnungsmittel wie Wasser, Öle und/oder Suspensionsmittel wie Polyethylenglykole oder dergleichen enthalten. Es können zusätzlich weitere Hilfsstoffe zugegeben werden, wie z.B. Konservierungsmittel, Geschmackskorregenzien und dergleichen.

Die Wirkstoffe können mit den pharmazeutischen Hilfs- und/oder Trägerstoffen in an sich bekannter Weise gemischt und formuliert werden. Zur Herstellung fester Arzneiformen können die Wirkstoffe beispielsweise mit den Hilfs- und/oder Trägerstoffen in üblicher Weise gemischt und naß oder trocken granuliert werden. Das Granulat oder Pulver kann direkt in Kapseln abgefüllt oder in üblicher Weise zu Tablettenkernen verpreßt werden. Diese können gewünschtenfalls in bekannter Weise dragiert werden.

Die Verbindungen der Formel I können auf an sich bekannte Weise nach den in der vorgenannten DE-OS 26 58 558 beschriebenen Verfahren oder analog zu diesen Verfahren hergestellt werden. Beispielsweise können Verbindungen der Formel I erhalten werden, indem man Verbindungen der allgemeinen Formel II
(siehe Formel II)
worin $R^1$, $R^2$ und $R^3$ obige Bedeutung besitzen, mit Säuren oder reaktionsfähigen Säurederivaten der allgemeinen Formel III
(siehe Formel III)
worin $R^4$ und $R^5$ obige Bedeutung besitzen und X Hydroxy oder eine reaktive Gruppe bedeutet, acyliert, und gewünschtenfalls freie Verbindungen der Formel I in ihre physiologisch verträglichen Säureadditionssalze überführt oder die Säureadditionssalze in die freien Verbindungen der Formel I überführt.

Die Umsetzung der Amine der Formel II mit den Säuren oder Säurederivaten der Formel III kann nach an sich zur Amidbildung üblichen Methoden durchgeführt werden. Als reaktionsfähige Säurederivate der Formel III kommen insbesondere Säurehalogenide, vorzugsweise -chloride, und Säureanhydride in Frage. Die Acylierung kann in einem unter den Reaktionsbedingungen inerten Lvsungsmittel gew|nschtenfalls in Gegenwart eines sdurebindenden Mittels durchgef|hrt werden. Als Lvsungsmittel eignen sich beispielsweise halogenierte Kohlenwasserstoffe wie Dichlormethan, aromatische Kohlenwasserstoffe wie Benzol, cyclische Dther wie Tetrahydrofuran oder Dioxan, Dimethylformamid oder Gemische dieser Lvsungsmittel. Als sdurebindende Mittel eignen sich anorganische Basen, insbesondere Alkalimetallhydroxide, oder organische Basen wie tertidre Niederalkylamine und Pyridine.

Die als Ausgangsverbindungen verwendeten 3,7-Diazabicylo[3,3,1]nonan-Verbindungen der Formel II sind aus den DE-OS 26 58 558 und DE-OS 37 22 134 bekannt und/oder kvnnen nach den in diesen Schriften beschriebenen Methoden oder analog zu den in diesen Schriften beschriebenen Methoden auf an sich bekannte Weise hergestellt werden.

Die nachfolgenden Beispiele sollen die Erfindung ndher erldutern, jedoch deren Umfang in keiner Weise beschrdnken.

Die folgenden Beispiele 1 bis 3 beschreiben erfindungsgemd_e pharmazeutische Zubereitungen enthaltend einen Wirkstoff der Formel I sowie die Herstellung solcher pharmazeutischen Zubereitungen.

Beispiel 1: Tabletten

Zusammensetzung:

| | |
|---|---|
| 3-(4-Chlorbenzoyl)-7-(n-butyl)-9,9-tetramethylen-3,7-diazabicyclo[3,3,1]nonan-monohydrochlorid | 20 Teile |
| Maisstdrke | 30 Teile |
| Lactose | 55 Teile |
| Kollidon $25^R$ | 5 Teile |
| Magnesiumstearat | 2 Teile |
| Talkum | 3 Teile |
| Gesamt | 115 Teile |

Herstellungsvorschrift:

Der Wirkstoff wird mit Maisstärke und feinpulvriger Lactose in einem Mischer vermischt. Die entstandene Mischung wird mit einer 20%-igen Lösung von Polyvinylpyrrolidon (Kollidon $25^R$ der Fa. BASF) in entmineralisiertem Wasser durchfeuchtet. Falls erforderlich wird weiteres entmineralisiertes Wasser hinzugefügt. Das feuchte Granulat wird durch ein 2 mm-Sieb passiert, bei 40 °C auf Horden getrocknet und anschließend durch ein 1 mm-Sieb (Frewitt-Maschine) passiert. Nach dem Vermischen des Granulates mit Magnesiumstearat und Talkum werden daraus Tabletten mit einem Gewicht von 115 mg gepreßt, so daß jede Tablette 20 mg Wirkstoff enthält.

Beispiel 2: Kapseln

Zusammensetzung:

| | |
|---|---|
| 3-(2,4-Dichlorbenzoyl)-7-methyl-9,9-pentamethylen-3,7-diazabicyclo[3,3,1]nonan-monohydrochlorid | 20 Teile |
| Maisstärke | 20 Teile |
| Lactose | 45 Teile |
| Kollidon $25^R$ | 3 Teile |
| Magnesiumstearat | 1,5 Teile |
| Aerosil $200^R$ | 0,5 Teile |
| Gesamt | 90 Teile |

Herstellungsvorschrift:

Der Wirkstoff wird mit Maisstärke und feinpulvriger Lactose in einem Mischer vermischt. Die entstandene Mischung wird mit einer 20%-igen Lösung von Polyvinylpyrrolidon (Kollidon $25^R$ der Fa. BASF) in entmineralisiertem Wasser durchfeuchtet. Falls erforderlich wird entmineralisiertes Wasser hinzugefügt. Das feuchte Granulat wird durch ein 1,6 mm-Sieb (Frewitt-Maschine) passiert, bei 40°C auf Horden getrocknet und anschließend durch ein 1 mm-Sieb (Frewitt) passiert. Nach dem Vermischen des Granulates mit Magnesiumstearat und hochdisperser Kieselsäure (Aerosil $200^R$ der Fa. Degussa) füllt man davon jeweils 90 mg mittels automatischer Kapselabfüllmaschine in Hartgelatinekapseln der Größe 4 ab, so daß jede Kapsel 20 mg Wirkstoff enthält.

Beispiel 3: Ampullen

Zusammensetzung (pro Ampulle):

| | |
|---|---|
| 3-(2,4-Dichlorbenzoyl)-7-(n-butyl)-9,9-tetramethylen-3,7-diazabicyclo[3,3,1]nonan-hydrochlorid | 5 mg |
| Natriumchlorid | 16 mg |
| Wasser für Injektionszwecke ad | 2,0 mg |

Herstellungsvorschrift:

Man löst Natriumchlorid in Wasser für Injektionszwecke auf, fügt den Wirkstoff hinzu und löst unter Rühren. Mit genügend Wasser für Injektionszwecke wird bis zum Endvolumen aufgefüllt. Der Ansatz wird durch ein Membranfilter 0,25 μ passiert. Man füllt in Braunglasampullen jeweils 2,15 ml ab und schmilzt sie zu. Bei 121°C wird 30 Min. lang mit Dampf sterilisiert. 2 ml Injektionslösung enthalten 5 mg Wirkstoff.

Die nachfolgenden Beispiele sollen die Herstellung der Verbindungen der Formel I näher erläutern.

Beispiel 4:

3-(4-Chlorbenzoyl)-7-(n-butyl)-9,9-tetramethylen-3,7-diazabicyclo[3,3,1]nonan.

Zu einer Lösung von 5,91 g 7-(n-Butyl)-9,9-tetramethylen-3,7-diazabicyclo[3,3,1]nonan in einer Mischung aus 80 ml Dichlormethan und 10 ml wäßriger Natriumhydroxidlösung wurden 4,38 g 4-Chlorbenzoylchlorid tropfenweise unter Rühren bei Eiskühlung zugegeben. Das Reaktionsgemisch wurde eine Stunde reagieren gelassen. Dann wurden 100 ml Wasser zugegeben, die organische Phase abgetrennt und die wäßrige Phase zweimal mit Dichlormethan extrahiert. Die vereinigten organischen Phasen wurden mit Magnesiumsulfat getrocknet und eingeengt. Es wurden 8,6 g 3-(4-Chlorbenzoyl)-7-(n-butyl)-9,9-tetramethylen-3,7-diazabicyclo[3,3,1]nonan als Öl erhalten, welches im Kühlschrank kristallisierte. Schmelzpunkt 105 bis 107°C.

Durch Umsetzung mit isopropanolischer Salzsäurelösung wurde die Titelverbindung in ihr Hydrochlorid mit einem Schmelzpunkt von 220 bis 230 °C überführt.

Nach der in Beispiel 4 beschriebenen Methode wurden auch die folgenden Verbindungen der Formel I erhalten.

| Bsp.Nr. | $R^1$ | $R^2$ | $R^3$ | $R^4$ | $R^5$ | Salz | Fp. in °C |
|---|---|---|---|---|---|---|---|
| 5 | n-$C_4H_9$- | $CH_3$- | $CH_3$- | H | H | 1 • HCl | 180-185 |
| 6 | n-$C_4H_9$- | $CH_3$- | $CH_3$- | 4-$NO_2$ | H | 1,5 • HTa | 97 |
| 7 | n-$C_4H_9$- | $CH_3$- | $CH_3$- | 4-Cl | H | 1 • HCl | 196-198 |
| 8 | n-$C_4H_9$ | $CH_3$- | $CH_3$- | 4-Br | H | B | 99-101 |
| 9 | n-$C_4H_9$- | $CH_3$- | $CH_3$- | 4-F | H | B | 63-66 |
| 10 | n-$C_4H_9$- | $CH_3$- | $CH_3$- | 4-CN | H | 1 • HCl | 246-250 |
| 11 | n-$C_4H_9$- | $CH_3$- | $CH_3$- | 4-$SO_2CH_3$ | H | 1,1 • HCl | 132-137 |
| 12 | n-$C_6H_{13}$- | $CH_3$- | $CH_3$- | 4-CN | H | 1,4 • HTa | am |
| 13 | n-$C_6H_{13}$- | $CH_3$- | $CH_3$- | 4-$SO_2CH_3$ | H | 1,4 • HTa | am |
| 14 | n-$C_4H_9$- | -$(CH_2)_4$- | | 4-CN | H | 1,4 • HTa | am |
| 15 | n-$C_4H_9$- | -$(CH_2)_4$- | | 4-Br | H | B | 106-108 |
| 16 | n-$C_4H_9$- | -$(CH_2)_4$- | | 4-F | H | B | 85 |
| 17 | n-$C_4H_9$- | -$(CH_2)_4$- | | 2-F | 4-F | 1 • HCl | 234 |
| 18 | n-$C_4H_9$- | -$(CH_2)_4$- | | 2-Cl | 4-Cl | 1,1 • HCl | 237-239 |
| n=normal, i=iso, Cyp=cyclopropyl, am=amorph, B=Base, HTa=Hydrogentartrat, HCl=Hydrochlorid | | | | | | | |

| Bsp.Nr. | $R^1$ | $R^2$ | $R^3$ | $R^4$ | $R^5$ | Salz | Fp. in °C |
|---|---|---|---|---|---|---|---|
| 19 | n-$C_4H_9$- | -$(CH_2)_4$- | | 4-$NO_2$ | H | 1 • HCl | 236-239 |
| 20 | n-$C_4H_9$- | -$(CH_2)_4$- | | 4-$SO_2F$ | H | 1 • HCl | 220 |
| 21 | n-$C_4H_9$- | -$(CH_2)_4$- | | 4-$CF_3$ | H | 1,4 • HTa | am |
| 22 | n-$C_4H_9$- | -$(CH_2)_4$- | | 4-$SO_2CH_3$ | H | 1,4 • HTa | am |
| 23 | n-$C_6H_{13}$- | -$(CH_2)_4$- | | 3-$NO_2$ | 5-$NO_2$ | B | 69.5 |
| 24 | i-$C_4H_9$- | -$(CH_2)_5$- | | 4-Cl | H | 1 • HCl | 260-264 |
| 25 | Cyp-$CH_2$- | $CH_3$- | $CH_3$- | 4-Cl | H | 1 • HCl | am. |
| 26 | n-$C_6H_{13}$- | $CH_3$- | $CH_3$- | 4-Cl | H | B | 230-240 |
| 27 | $CH_3$- | -$(CH_2)_5$- | | 2-Cl | 4-Cl | 1 • HCl | 255-257 |
| 28 | n-$C_4H_9$- | -$(CH_2)_4$- | | 2-$NO_2$ | 4-Cl | 1 • HCl | 156 |
| 29 | n-$C_4H_9$- | n-$C_3H_7$- | $CH_3$- | 3-Cl | H | 1 • HCl | 236-238 |
| 30 | $C_2H_5$- | -$(CH_2)_4$- | | 2-$CF_3$ | 5-$CF_3$ | B | 99-101 |

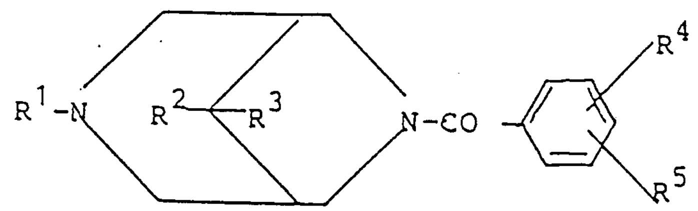

I

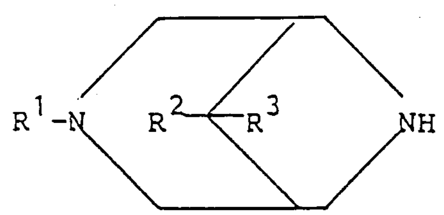

II

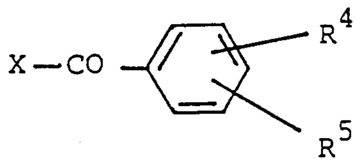

III

## Patentansprüche

1. Verwendung von 3-Benzoyl-3,7-diazabicyclo[3,3,1]nonan-Verbindungen der allgemeinen Formel I

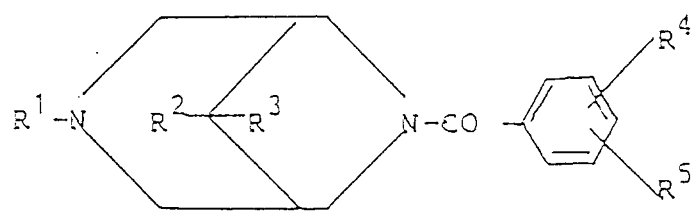

I

worin

| | |
|---|---|
| $R^1$ | eine Alkylgruppe mit 1-6 Kohlenstoffatomen oder eine Cycloalkylalkylgruppe mit 4-7 Kohlenstoffatomen bedeutet, |
| $R^2$ | Alkyl mit 1 - 4 Kohlenstoffatomen bedeutet und |
| $R^3$ | Alkyl mit 1 - 4 Kohlenstoffatomen bedeutet oder |
| $R^2$ und $R^3$ | gemeinsam eine Alkylenkette mit 3-6 Kohlenstoffatomen bilden, |
| $R^4$ | für Wasserstoff, Halogen, Cyano, Nitro, Trifluormethyl oder die $R^6$-$SO_2$-Gruppe, worin $R^6$ Fluor oder |

| | |
|---|---|
| | Alkyl mit 1 bis 4 Kohlenstoffatomen bedeutet, steht, und |
| $R^5$ | Wasserstoff, Halogen, Trifluormethyl oder Nitro bedeutet, |

und deren physiologisch verträglichen Säureadditionssalzen zur Herstellung von pharmazeutischen Zubereitungen zur Behandlung von Herzrhythmusstörungen in größeren Säugetieren und Menschen.

**2.** Verwendung gemäß Anspruch 1, dadurch gekennzeichnet, daß Verbindungen der Formel I eingesetzt werden, worin $R^1$ eine Alkylgruppe mit 3 - 5 Kohlenstoffatomen bedeutet, $R^2$ und $R^3$ gemeinsam eine Alkylenkette mit 4 - 5 Kohlenstoffatomen bilden, $R^4$ Halogen, bedeutet und $R^5$ Wasserstoff oder Halogen bedeutet.

## Claims

**1.** Use of 3-benzoyl-3,7-diazabicyclo[3.3.1]-nonane compounds of the general formula I

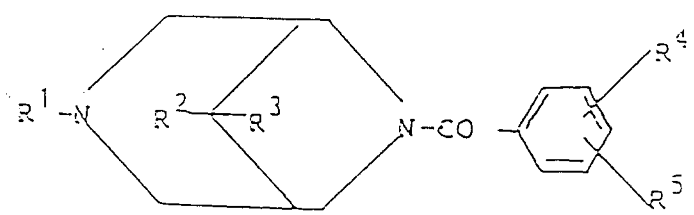

I

in which

| | |
|---|---|
| $R^1$ | is an alkyl group having 1 - 6 carbon atoms or a cycloalkylalkyl group having 4 - 7 carbon atoms, |
| $R^2$ | is alkyl with 1 - 4 carbon atoms and |
| $R^3$ | is alkyl with 1 - 4 carbon atoms or |
| $R^2$ and $R^3$ | together form an alkylene chain having 3 - 6 carbon atoms, |
| $R^4$ | is hydrogen, halogen, cyano, nitro, trifluoromethyl or the $R^6$-$SO_2$- group in which $R^6$ is fluorine or alkyl with 1 - 4 carbon atoms, and |
| $R^5$ | is hydrogen, halogen, trifluoromethyl or nitro, |

and their Physiologically tolerable acid addition salts for the production of pharmaceutical preparations for the treatment of cardiac arrhythmias in larger mammals and humans.

**2.** Use according to Claim 1, characterized in that compounds of the formula I are employed in which $R^1$ is an alkyl group having 3 - 5 carbon atoms, $R^2$ and $R^3$ together are an alkylene chain having 4 - 5 carbon atoms, $R^4$ is halogen and $R^5$ is hydrogen or halogen.

## Revendications

**1.** Utilisation de dérivés du 3-benzoyl-3,7-diazabicyclo[3,3,1] nonane, de formule générale I :

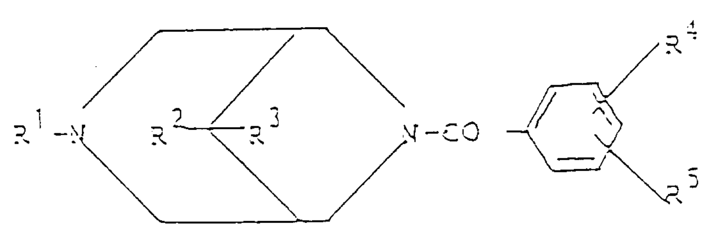

I

dans laquelle :

| | |
|---|---|
| $R^1$ | désigne un groupe alkyle ayant 1 à 6 atomes de carbone ou un groupe cycloalkylalkyle ayant 4 à 7 atomes de carbone; |

| | |
|---|---|
| $R^2$ | désigne un groupe alkyle ayant 1 à 4 atomes de carbone, et |
| $R^3$ | désigne un groupe alkyle ayant 1 à 4 atomes de carbone ou bien, |
| $R^2$ et $R^3$ | forment ensemble une chaîne alkylène ayant 3 à 6 atomes de carbone, |
| $R^4$ | représente un atome d'hydrogène ou d'halogène, un groupe cyano, nitro, trifluorométhyle ou le groupe $R^6$-$SO_2$-, dans lequel $R^6$ représente le fluor ou un groupe alkyle ayant 1 à 4 atomes de carbone, et |
| $R^5$ | représente un atome d'hydrogène, un atome d'halogène, un groupe trifluorométhyle ou nitro |

et de leurs sels d'addition d'acide physiologiquement tolérables pour produire des préparations pharmaceutiques pour le traitement de troubles du rythme cardiaque chez les gros mammifères et les êtres humains.

**2.** Utilisation selon la revendication 1, caractérisée en ce qu'on utilise des composés de formule I, dans laquelle $R^1$ désigne un groupe alkyle ayant 3 à 5 atomes de carbone; $R^2$ et $R^3$ forment ensemble une chaîne alkylène ayant 4 à 5 atomes de carbone; $R^4$ représente un atome d'halogène et $R^5$ représente un atome d'hydrogène ou d'halogène.